# EUROPÄISCHE PATENTANMELDUNG

(11) **EP 1 570 877 A1**
(43) Veröffentlichungstag der Anmeldung: **07.09.2005**
(21) Anmeldenummer: 05004698.6
(22) Anmeldetag: 03.03.2005
(51) Int. Cl.: A61M 15/00

(54) **Zählvorrichtung für mit Vorspannung arbeitende Sprühstoss-Dosisinhalatoren**

(30) Priorität: 03.03.2004 DE 102004010393
(71) Anmelder: Fujisawa Deutschland GmbH, 81673 München (DE)
(72) Erfinder: Ludwig, Ralf, Dr., 82110 Germering (DE); Busch, Heinrich, 81737 München (DE); Widderich, Carsten, Central, Hong Kong (CN); Leung, Stephen, Fotan, Shatin, NT, Hong Kong (CN)
(74) Vertreter: HOFFMANN EITLE

(57) **Zusammenfassung**

Die erfindungsgemäße Zählvorrichtung eignet sich speziell für einen Vorspannungsmechanismus aufweisende Sprühstoß-Dosisinhalatoren (1, 2), bei denen mit Hilfe des Vorspannungsmechanismus (8) eine die Abgabe eines Sprühstoßes vorbereitende Vorspannung erzeugt wird. Die Zählvorrichtung umfasst ein Zählwerk (103) für die schrittweise Zählung von Sprühstößen des Sprühstoß-Dosisinhalators, einen Halterungsabschnitt (101) für die lösbare Halterung der Zählvorrichtung (100) an dem Gehäuse (2) des Dosisinhalators; einen Öffnungsbereich (102) für die Aufnahme eines durch einen Benutzer des Sprühstoß-Dosisinhalators zu betätigenden Teils (9) des Vorspannungsmechanismus (8), in dem der durch einen Benutzer des Sprühstoß-Dosisinhalators zu betätigende Teil (9) des Vorspannungsmechanismus (8) bei an dem Sprühstoß-Dosisinhalator gehalterter Zählvorrichtung (100) angeordnet ist; und eine Zählschrittschalteinrichtung (107) für die schrittweise Weiterschaltung des Zählwerks (103), die in dem Öffnungsbereich (102) derart angeordnet ist, dass bei jeder Betätigung der durch einen Benutzer des Sprühstoß-Dosisinhalators zu betätigenden Teil (9) des Vorspannungsmechanismus (8) auf die Zählschrittschalteinrichtung eine Weiterschaltung des Zählwerks auslösend einwirkt.

## Beschreibung

Die Erfindung betrifft eine Zählvorrichtung für einen Vorspannungsmechanismus aufweisende Sprühstoß-Dosisinhalatoren, bei denen mit Hilfe einer Vorspannungsaufbringungseinrichtung eine die Abgabe eines Sprühstoßes vorbereitende Vorspannung erzeugt wird.

Sprühstoß-Dosisinhalatoren, oft auch als MDI (Metered Dose Inhaler) bezeichnet, sind aufgrund ihrer Größe und einfachen Handhabung als tragbares Inhalationstherapiegerät in vielen Anwendungsfällen einsetzbar. Sie umfassen einen Medikamentbehälter, in dem das Medikament zusammen mit einem Treibmittel aufbewahrt wird, und einen Sprühspender, in den der Medikamentbehälter eingesetzt wird und der unter anderem eine Düse und ein Mundstück umfasst. Wird der Medikamentbehälter in Bezug auf den Sprühspender verschoben, beispielsweise indem der Benutzer den Medikamentbehälter in den Sprühspender hineindrückt, wird ein Sprühstoß ausgelöst, der über die Düse und das Mundstück dem Benutzer als einatembares Aerosol dargeboten wird.

In der Regel kann aber der Füllstand des Medikamentbehälters von außen nicht ohne weiteres überprüft werden. Deshalb sind in kritischen Anwendungsfällen zuverlässige Zählvorrichtungen erforderlich, mit denen die Anzahl der abgegebenen Sprühstöße bzw. der noch abgebbaren Sprühstöße ermittelt und dem Benutzer angezeigt werden. Verschiedene Lösungen sind vorgeschlagen worden.

So beschreibt EP 0 752 895 B einen eigenständigen Zähler für Sprühstoß-Dosisinhalatoren, der auf den Medikamentbehälter des Dosisinhalators aufsteckbar ist und einen Drucksensor aufweist, mit dessen Hilfe bei jeder Betätigung des Dosisinhalators eine elektrische Zähleinrichtung weitergeschaltet wird, deren aktueller Zählwert in einer Zählwertanzeige angezeigt wird. Der Zähler ist an dem Behälter über eine federnde Schürze befestigt, die im aufgesteckten Zustand an dem Behälter anliegt, so dass der Bereich des Druckssensors und der Zählanzeige über dem Boden des Behälters und damit über dem Bereich des Behälters angeordnet ist, auf den durch einen Benutzer eine Druckkraft zur Betätigung des Dosisinhalators aufgebracht wird. Von besonderer Bedeutung bei diesem Zähler ist die Empfindlichkeit des Drucksensors, mit der der aufgebrachte Druck erfasst wird. Ist die Empfindlichkeit zu groß, werden leichte Berührungen des Drucksensors als Betätigungen gewertet und das Zählwerk weitergeschaltet, obwohl kein Sprühstoß ausgelöst wurde. Ist die Empfindlichkeit zu klein, werden Sprühstöße nicht gezählt, obwohl eine einen Sprühstoß auslösende Druckkraft aufgebracht worden ist. Entsprechend aufwändig sind die in EP 0 752 895 B beschriebenen Maßnahmen zur Sicherstellung der Zählgenauigkeit.

Ein ähnliches auf den Boden des Medikamentbehälters eines Dosisinhalators aufsteckbares Zählwerk ist aus WO 86/02775 A bekannt; jedoch handelt es sich hier um eine mechanische Lösung, bei der mehrere Ziffernscheiben weitergeschaltet werden. Mechanische Lösungen haben stets den Nachteil der regelmäßig hohen Herstellungskosten gepaart mit einer auf der Vielzahl der mechanischen Teile beruhenden höheren Störungsanfälligkeit.

Ein anderes elektrisches Zählwerk ist aus EP 0 448 204 bekannt, dass am Sprühspender eines Dosisinhalators angebracht ist. Wird ein Sprühstoß ausgelöst, indem der Benutzer den Medikamentbehälter in den Sprühspender hineindrückt, betätigt der Medikamentbehälter bei seiner Bewegung einen Schalthebel, der im Inneren des Sprühspenders in den Bewegungsbereich des Medikamentbehälters hineinragt und mit einem Schalter in einem elektrischen Zählwerk verbunden ist, wodurch bei jeder Betätigung des Dosisinhalators das Zählwerk weiterschaltet wird. Der Schalthebel ragt durch eine kleine Wandöffnung in den Sprühspender hinein. Es ist zwar in EP 0 448 204 erwähnt, dass das Zählwerk auswechselbar sein soll, jedoch weisen Sprühspender üblicherweise nicht die für den Durchtritt des Schalthebels erforderliche Wandöffnung auf, so dass eine universelle Einsetzbarkeit nicht gegeben ist. Außerdem ist auch bei diesem Zählwerk nicht in ausreichendem Maße gewährleistet, dass jede Betätigung des Sprühverneblers erfasst wird, da kurze Bewegungen des Medikamentbehälters einen falschen Zählimpuls auslösen können.

Grundsätzlich ist bei Dosisinhalatoren zu gewährleisten, dass die Betätigung in der Art erfolgt, dass ein Sprühstoß dann durch den Benutzer von Hand ausgelöst wird, wenn er einatmet. Das bedeutet, dass der Benutzer seine Atmung und die Abgabe des Sprühstoßes genau koordinieren muss, was oftmals aber Schwierigkeiten bereitet. Eine Abhilfe bieten in diesem Zusammenhang mit Vorspannung arbeitenden Sprühstoß-Dosisinhalatoren, bei denen die Abgabe des Sprühstoßes durch eine automatische-Atemzugsteuerung ausgelöst wird.

Bei diesen Dosisinhalatoren, beispielsweise Spannhebeldosisinhalatoren sind die bekannten Zählwerke aber nicht einsetzbar, denn diese Sprühstoß-Dosisinhalatoren zeichnen sich zur Erreichung einer hohen Dosisgenauigkeit dadurch aus, dass der Erzeugung des Sprühstoßes eine Aktivierung des Dosisinhalators vorausgeht und der eigentliche Sprühstoß erst beim Einatmen des Benutzers, in der Regel ohne weiteres Zutun des Benutzers, allein durch die Atemzugsteuerung ausgelöst wird. Dazu drückt ein in Aktivierungsstellung gebrachter Spannhebelmechanismus auf den Medikamentbehälter in dem Spannhebeldosisinhalator in Richtung auf die Düsen-/Mundstückeinheit, wodurch auf den Medikamentbehälter zum Beispiel mittels einer Feder eine definierte Vorspannungskraft einwirkt, die für die Abgabe einer genauen Dosis in Form eines Sprühstoßes abgestimmt ist. Jedoch wird der Sprühstoß nicht sofort abgegeben, da in der Düseneinheit die den Sprühstoß frei gebende Bewegung des Medikamentbehälters verhindert wird. Erst wenn durch den Fluss der Atemluft beim Einatmen des Benutzers eine Sperre aus einer verriegelnden Stellung weg bewegt wird, ist die Bewegung des Medikamentbehälters in dem Dosisinhalator möglich, wodurch die Abgabe des Sprühstoßes erfolgt.

Der für die Aufbringung der Vorspannung erforderliche Spannhebel verhindert den Einsatz des drucksensorgesteuerten Zählwerks der eingangs beschriebenen Art. Zudem ist mit der Druckkraftaufbringung im vorgespannten Zustand nicht zwingend die Abgabe eines Sprühstoßes verbunden, da letzterer erst durch das Einatmen des Benutzers ausgelöst wird. Schließlich eignet sich das Drucksensorzählwerk nicht für Vorspannungsdosisinhalatoren, da der Medikamentbehälter bei diesen Dosisinhalatoren in einem geschlossenen Gehäuse angeordnet ist.

Ein Zählwerk mit Schalthebel, wie oben beschrieben, ist zwar grundsätzlich bei einen Vorspannungsmechanismus aufweisenden Dosisinhalatoren einsetzbar, jedoch sind die Auswechselbarkeit und insbesondere die nachträgliche Einsetzbarkeit wegen der erforderlichen Wandöffnung nicht in jedem Fall gegeben. Vielmehr erfordert dieses Zählwerk auch bei Spannhebeldosisinhalatoren eine spezielle Anpassung.

Vor diesem Hintergrund besteht das von der Erfindung zu lösende Problem darin, ein Zählwerk aufzuzeigen, das bei einen Vorspannungsmechanismus aufweisenden Dosisinhalatoren, insbesondere Spannhebeldosisinhalatoren universell, d.h. auswechselbar eingesetzt werden kann. Dabei soll die Lösung kostengünstig herstellbar sein und eine für Vorspannungdosisinhalatoren ausreichende Zählgenauigkeit und Zuverlässigkeit aufweisen.

Dieses Problem wird gelöst durch eine Zählvorrichtung für einen Vorspannungsmechanismus aufweisende Sprühstoß-Dosisinhalatoren, bei denen mit Hilfe des Vorspannungsmechanismus eine die Abgabe eines Sprühstoßes vorbereitende Vorspannung erzeugbar ist, mit einem Zählwerk für die schrittweise Zählung von Sprühstößen des Sprühstoß-Dosisinhalators; einem Halterungsabschnitt für die lösbare Halterung der Zählvorrichtung an dem Gehäuse des Dosisinhalators; einem Öffnungsbereich für die Aufnahme eines durch einen Benutzer des Sprühstoß-Dosisinhalators zu betätigenden Teil des Vorspannungsmechanismus, in dem der durch einen Benutzer des Sprühstoß-Dosisinhalators zu betätigende Teil des Vorspannungsmechanismus bei an dem Sprühstoß-Dosisinhalator gehaltertem Zählwerk angeordnet ist; und einer Zählschrittschalteinrichtung für die schrittweise Weiterschaltung des Zählwerks, die in dem Öffnungsbereich derart angeordnet ist, dass bei jeder Betätigung der durch einen Benutzer des Sprühstoß-Dosisinhalators zu betätigenden Teil des Vorspannungsmechanismus auf die Zählschrittschalteinrichtung eine Weiterschaltung des Zählwerks auslösend einwirkt.

Die erfindungsgemäße Lösung zeichnet sich zunächst dadurch aus, dass die Halterung des Zählers an dem Gehäuse des Dosisinhalators erfolgt, so dass die Unterbringung des Medikamentbehälters in dem Gehäuse kein Hindernis für den Einsatz des erfindungsgemäßen Zählers darstellt. Durch die erfindungsgemäße Öffnung wird erreicht, dass der zu betätigende Teil des Vorspannungsmechanismus, zum Beispiel der Spannhebel einerseits weiter zugänglich ist und andererseits die Anbringung des Zählers nicht verhindert. Die Anordnung des Betätigungsteils in der Öffnung des Zählergehäuses ist die Grundlage für einen weiteren die Erfindung auszeichnenden Aspekt, nämlich die unmittelbare Einbeziehung des Betätigungsteils in den Zählvorgang. Dazu wird der Zählschrittschalter erfindungsgemäß in dem Öffnungsbereich angeordnet, wodurch sichergestellt wird, dass eine Betätigung des Vorspannungsmechanismus zu einer Zählung führt.

Vorteilhafte Ausgestaltungen der erfindungsgemäßen Zählvorrichtung ergeben sich aus den Unteransprüchen sowie aus der folgenden Beschreibung, in der unter Bezugnahme auf die Figuren ein Ausführungsbeispiel erläutert wird.

In den Figuren zeigt:
- Fig. 1: eine schematische Darstellung eines Sprühstoß-Dosisinhalators mit Vorspannmechanismus;
- Fig. 2: eine schematische, teilweise geschnittene Darstellung einer erfindungsgemäßen Zählvorrichtung an dem Sprühstoß-Dosisinhalator mit Vorspannmechanismus gemäß Figur 1;
- Fig. 3: eine erste perspektivische Darstellung der einfindungsgemäßen Zählvorrichtung aus Figur 2; und
- Fig. 4: eine zweite perspektivische Darstellung der einfindungsgemäßen Zählvorrichtung aus Figur 2 bzw. 3.

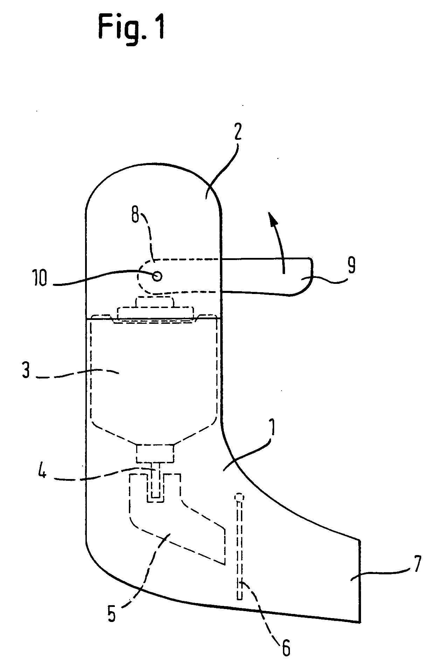

Fig. 1 zeigt einen Sprühstoß-Dosisinhalator, der einen Vorspannungsmechanismus aufweist. Der Sprühstoß-Dosisirihalator gemäß Fig. 1 umfasst ein Gehäuse, das einen den Sprühspender bildenden ersten Teil 1 und einen den Vorspannungsmechanismus aufnehmenden zweiten Teil 2 umfasst. In dem Gehäuse wird ein Medikamentenbehälter 3, der in Fig. 1 gestrichelt dargestellt ist, aufgenommen, so dass dessen Medikamentabgaberöhrchen 4 in einer Düse 5 des Sprühspenders 1 angeordnet ist. Vor der Düse 5 befindet sich eine Atemzugklappe 6, die Teil einer nicht weiter dargestellten Atemzugsteuerung ist und dafür sorgt, dass beim Einatmen des Benutzers durch das Mundstück 7 ein Sprühstoß ausgelöst wird. Ist der Medikamentbehälter 3 leer, wird der zweite Gehäuseteil 2 vom ersten Gehäuseteil 1 getrennt, beispielsweise abgeschraubt, so dass es möglich ist, den leeren Medikamentbehälter 3 zu entnehmen und einen vollen Medikamentbehälter 3 einzusetzen.

Für die Bereitstellung einer dosisgenauen Medikamentenmenge wird mit Hilfe des Vorspannungsmechanismus 8, der in Fig. 1 nur beispielhaft und schematisch dargestellt ist, eine Druckkraft auf den Medikamentbehälter 3 ausgeübt, wenn der Spannhebel 9 um seine Drehachse 10 in eine Aktivierungsstellung geschwenkt wird, die beispielsweise erreicht ist, wenn der Spannhebel 9 senkrecht zu der in Fig. 1 gezeigten Ruhestellung liegt. Befindet sich der Spannhebel 9 in der Aktivierungsstellung, und atmet der Benutzer am Mundstück 7 ein, wird die Aktivierungsklappe 6 bewegt, so dass der Auslösemechanismus (nicht dargestellt) der Atemzugsteuerung die Abgabe des Sprühstoßes über die Düse 5 auslöst. Danach muss der Spannhebel 9 aus der Aktivierungsstellung wieder in die Ruhestellung gemäß Fig. 1 bewegt werden, um erneut in die Aktivierungsstellung gebracht werden zu können, so dass der Vorspannungsmechanismus wieder eine für die Abgabe eines dosisgenauen Sprühstoßes erforderliche Druckkraft auf den Medikamentbehälter 3 ausübt.

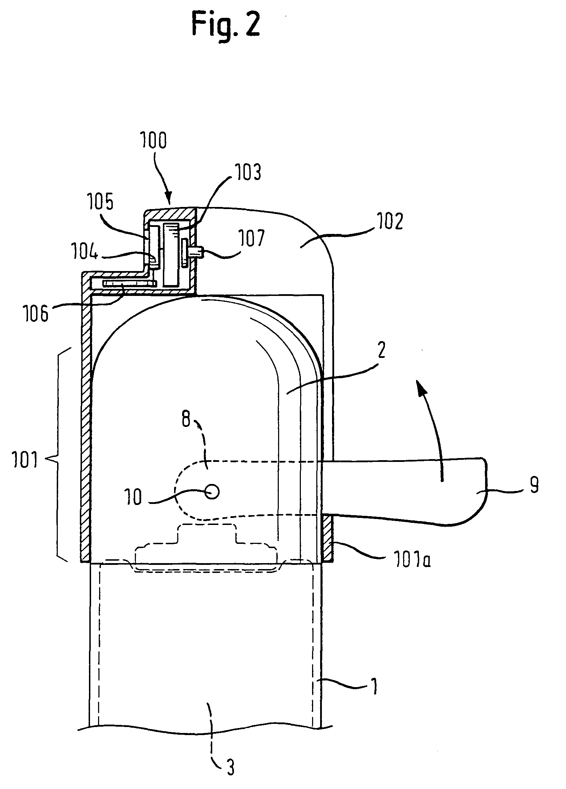

Fig. 2 zeigt neben dem Sprühstoß-Dosisinhalator eine erfindungsgemäße Zählvorrichtung 100, die auf den Sprühstoß-Dosisinhalator aufgesteckt ist. Dazu besitzt das Gehäuse der Zählvorrichtung 100 einen Halterungsabschnitt 101, der den Sprühstoß-Dosisinhalator zum Teil in sich aufnimmt und so die erfindungsgemäße Zählvorrichtung 100 an dem Sprühstoß-Dosisinhalator 1 und 2 befestigt. In dem Ausführungsbeispiel ist der Halterungsabschnitt 101 rohrförmig, da er in Form eines Mantelabschnitts eines Kreiszylinders realisiert und an die Form, insbesondere den Durchmesser des zweiten Gehäuseteils 2 des Dosisinhalators angepasst ist. Aufgrund der Passung wird die erfindungsgemäße Zählvorrichtung 100 sicher aber entfernbar an dem Dosisinhalator gehaltert, wenn die Zählvorrichtung 100 auf den Dosisinhalator aufgesteckt ist.

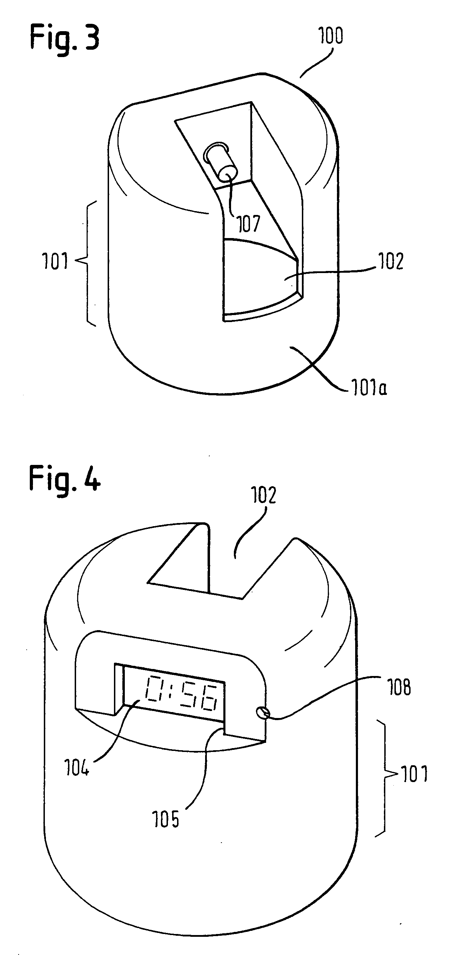

Der für die Haltung vorgesehene rohrförmige Abschnitt 101 des Zählwerkgehäuses ist auch in Fig. 3 erkennbar, die eine perspektivische Darstellung der Zählvorrichtung 100 gemäß Fig. 2 zeigt.

Sowohl in Fig. 2 als auch in Fig. 3 ist eine Öffnung 102 in dem Gehäuse der Zählvorrichtung 100 erkennbar. Die Öffnung 102 bildet einen größeren geöffneten Bereich in dem Gehäuse und dient zur Aufnahme des Spannhebels 9, wie sich aus Fig. 2 ergibt. Der Öffnungsbereich 102 ist derart ausgestaltet, dass der Spannhebel 9 aus der in Fig. 2 gezeigten Ruhestellung in die Aktivierungsstellung bewegt werden kann. Der dafür erforderliche Öffnungsbereich 102 in der Zählvorrichtung 100 gewährleistet nicht nur die freie Bewegung des Spannhebels von der einen in die andere Stellung und zurück, sondern auch das Aufstecken bzw. Entfernen der erfindungsgemäßen Zählvorrichtung 100 auf den Dosisinhalator.

Bei dem hier gezeigten Ausführungsbeispiel muss der Spannhebel 9 für das Aufstecken bzw. Entfernen der Zählvorrichtung 100 zumindest soweit aus der Ruhestellung gemäß Fig. 2 bewegt werden, dass die Zählvorrichtung 100 aufgesteckt werden kann. Denn wie sich aus Fig. 2 ergibt, liegt im aufgesteckten Zustand ein Teil 101a des Halterungsabschnitts 101 unter dem in Ruhestellung befindlichen Spannhebel 9. Auf diese Weise ist gewährleistet, dass sich eine aufgesteckte Zählvorrichtung 100 gemäß der Erfindung nicht versehentlich von dem Sprühstoß-Dosisinhalator abrutscht.

Bei dem in Fig. 2 und 3 gezeigten Ausführungsbeispiel einer erfindungsgemäßen Zählvorrichtung 100 ist vorteilhaft, dass sich das Gehäuse der Zählvorrichtung 100 lediglich bis zur Grenze zwischen dem ersten und dem zweiten Teil 1 bzw. 2 des Dosisinhalators erstreckt. Dadurch kann der zweite Teil 2 vom ersten Teil 1 des Dosisinhalators zusammen mit der gehalterten Zählvorrichtung 100 abgenommen, beispielsweise abgeschraubt werden, um den Medikamentbehälter 3 im Inneren des Dosisinhalators auszutauschen. Auf diese Weise ist gewährleistet, dass ein leerer Medikamentbehälter 3 auch bei aufgesteckter Zählvorrichtung 100 aus dem Sprühstoß-Dosisinhalator entnommen und ein neuer Medikamentbehälter 3 eingesetzt werden kann.

In dem Gehäuse der erfindungsgemäßen Zählvorrichtung 100 ist ein elektronisches Zählwerk 103 vorgesehen, dessen Zählerstand kontinuierlich in einer Zählwertanzeige 104 angezeigt wird. Durch eine Öffnung 105 im Gehäuse der Zählvorrichtung 100 kann der Zählwert vom Benutzer jederzeit abgelesen werden. Zählwerk 103 und Zählwertanzeige 104 sind für die Anzeige des Zählwerts miteinander verbunden. Ferner ist eine Stromversorgung 106 vorgesehen, die sowohl das elektronische Zählwerk 103 als auch die Anzeige 104 mit ausreichend Energie versorgt. Als Stromversorgung 106 kommt beispielsweise eine Knopfzelle in Betracht, die sämtliche elektrischen Bauteile über einen ausreichend langen Zeitraum mit Energie versorgen kann.

Erfindungsgemäß ist in der Zählvorrichtung 100 eine Schalteinrichtung 107 vorgesehen, die mit dem Zählwerk 103 derart verbunden ist, dass eine Betätigung der Schalteinrichtung 107 ein Weiterschalten des Zählwerks 103 und damit ein Herauf- oder Herabzählen des Zählwerts auslöst, der in der Anzeige 104 angezeigt wird. Die Schalteinrichtung 107 ist derart angeordnet, dass sie zur Betätigung in den Öffnungsbereich 102 hineinragt und durch einen in die Aktivierungsstellung gebrachten Spannhebel 9 betätigt wird. Im einfachsten Fall handelt es sich bei der Schalteinrichtung 107 um einen Taster, dessen Taststift durch eine Öffnung des Gehäuses der Zählvorrichtung 100 in den Öffnungsbereich 102 in unmittelbarer Nähe der Aktivierungsposition des Spannhebels 9 hineinragt, so dass im letzten Abschnitt der Bewegung des Spannhebels in die Aktivierungsstellung ein Schaltimpuls ausgelöst wird. Alternativ kann beispielsweise auch eine IR-Lichtschranke vorgesehen sein, deren Detektionsachse nahe und parallel zu dem Oberflächenabschnitt verläuft, in dem bei dem hier gezeigten Ausführungsbeispiel der Taster angeordnet ist.

Wie sich aus Fig. 2 und Fig. 3 ergibt, ist der Taster 107 in einem Oberflächenabschnitt der Wand der Öffnung 102 angeordnet, der in das Innere des Gehäuses der Zählvorrichtung 100 zurückspringt. Dadurch werden unbeabsichtigte Berührungen und Zählungen verhindert.

Fig. 4 zeigt die erfindungsgemäße Zählvorrichtung 100 ähnlich wie Fig. 3 in einer perspektivischen Ansicht, jedoch von der anderen Seite. In Fig. 4 ist die Zählwertanzeigeöffnung 105 und die Zählwertanzeige 104 erkennbar. Ferner ist in Fig. 4 auch der erfindungsgemäße Halterungsabschnitt 101 und der erfindungsgemäße Öffnungsbereich 102 dargestellt.

Zusätzlich zeigt Fig. 4 eine Rückstellöffnung 108, die Zugang zu einem Rückstelltaster des Zählwerks 103 gewährleistet, so dass das Zählwerk 103 nachdem Austausch des Medikamentbehälters 3 mit einem spitzen Gegenstand auf Null oder einen anderen definierten Anfangswert zurückgestellt werden kann.

Das Gehäuse der erfindungsgemäßen Zählvorrichtung 100 kann aus einem transparenten Kunststoff hergestellt werden, so dass eine spezielle Farbgebung des in der Zählvorrichtung aufgenommenen Teils des Sprühstoß-Dosisinhalators durchscheinend sichtbar bleibt. Alternativ kann das Gehäuse der erfindungsgemäßen Zählvorrichtung 100 aus einem farbigen Kunststoff gefertigt werden, wobei in diesem Fall Farben vorzugsweise eingesetzt werden, die gängigen Farbkodierungen an Sprühstoß-Dosisinhalatoren entsprechen.

## Patentansprüche

1. Zählvorrichtung für einen Vorspannungsmechanismus aufweisende Sprühstoß-Dosisinhalatoren (1, 2), bei denen mit Hilfe des Vorspannungsmechanismus (8) eine die Abgabe eines Sprühstoßes vorbereitende Vorspannung erzeugbar ist, mit
- einem Zählwerk (103) für die schrittweise Zählung von Sprühstößen des Sprühstoß-Dosisinhalators;
- einem Halterungsabschnitt (101) für die lösbare Halterung der Zählvorrichtung (100) an dem Gehäuse (2) des Dosisinhalators;
- einem Öffnungsbereich (102) für die Aufnahme eines durch einen Benutzer des Sprühstoß-Dosisinhalators zu betätigenden Teils (9) der Vorspannungsmechanismus (8), in dem der durch einen Benutzer des Sprühstoß-Dosisinhalators zu betätigende Teil (9) des Vorspannungsmechanismus (8) bei an dem Sprühstoß-Dosisinhalator gehalterter Zählvorrichtung (100) angeordnet ist; und
- einer Zählschrittschalteinrichtung (107) für die schrittweise Weiterschaltung des Zählwerks (103), die in dem Öffnungsbereich (102) derart angeordnet ist, dass bei jeder Betätigung der durch einen Benutzer des Sprühstoß-Dosisinhalators zu betätigenden Teil (9) des Vorspannungsmechanismus (8) auf die Zählschrittschalteinrichtung eine Weiterschaltung des Zählwerks auslösend einwirkt.

2. Zählvorrichtung nach Anspruch 1, **dadurch gekennzeichnet, dass** der Halterungsabschnitt (101) an einen Teil (2) des Gehäuses des Sprühstoß-Dosisinhalators zur Halterung der Zählvorrichtung (100) an dem Sprühstoß-Dosisinhalator formangepasst ist.

3. Zählvorrichtung nach Anspruch 2, **dadurch gekennzeichnet, dass** der Halterungsabschnitt (101) ein Mantelabschnitt eines Kreiszylinders ist, dessen Innendurchmesser für die halternde Aufnahme eines Gehäuseteils (2) mit kreisförmigem Querschnitt angepasst ist.

4. Zählvorrichtung nach Anspruch 1, 2 oder 3, **dadurch gekennzeichnet, dass** die Zählschrittschalteinrichtung (107) an dem Öffnungsbereich (102) derart angeordnet ist, dass nur bei einer eine definierte Vorspannung erzielenden Stellung des durch einen Benutzer des Sprühstoß-Dosisinhalators zu betätigenden Teils (9) des Vorspannungsmechanismus (8) eine Weiterschaltung des Zählwerks (103) der Zählvorrichtung (100) bewirkt wird.

5. Zählvorrichtung nach Anspruch 1, 2, 3 oder 4, **dadurch gekennzeichnet, dass** die Zählschrittschalteinrichtung (107) an einem in das Innere der Zählvorrichtung verlaufenden Wandabschnitt des Zählvorrichtungsgehäuse angeordnet ist.

6. Zählvorrichtung nach Anspruch 5, **dadurch gekennzeichnet, dass** die Zählschrittschalteinrichtung (107) ein Taster ist.

7. Zählvorrichtung nach Anspruch 6, **dadurch gekennzeichnet, dass** der Taststift des Tasters (107) durch eine Öffnung in der Gehäusewand der Zählvorrichtung (100) in den Öffnungsbereich (102) ragt.

8. Zählvorrichtung nach der vorangegangenen Ansprüche 1 bis 5, **dadurch gekennzeichnet, dass** eine IR-Lichtschranke ist.

9. Zählvorrichtung nach der vorangegangenen Ansprüche, **dadurch gekennzeichnet, dass** eine Zählwertanzeige (104) für die Anzeige des Zählerstands des Zählwerks (103) vorgesehen ist.

10. Zählvorrichtung nach der vorangegangenen Ansprüche, **dadurch gekennzeichnet, dass** eine Rückstelleinrichtung (108) für die Rückstellung des Zählwerks (103) auf einen Ausgangszählwert, insbesondere auf Null oder die Anzahl der maximal abgebbaren Sprühstöße vorgesehen ist.

11. Zählvorrichtung nach der vorangegangenen Ansprüche, **dadurch gekennzeichnet, dass** das Gehäuse der Zählvorrichtung (100) aus einem transparenten oder einem farbigen Kunststoff hergestellt ist.
